# FASCICULE DE BREVET EUROPEEN

(11) **EP 2 026 706 B1**
(45) Date de publication et mention de la délivrance du brevet: **18.11.2015**
(21) Numéro de dépôt: 07788855.0
(22) Date de dépôt: 08.06.2007
(51) Int. Cl.: A61B 17/72

(54) **DISPOSITIF D'ALLONGEMENT INTRACORPOREL A VIS TRAVAILLANT EN TRACTION**
VORRICHTUNG FÜR INTRAKORPORALE DEHNUNG MIT ZUGSCHRAUBEN
DEVICE FOR INTRABODY EXTENSION WITH SCREWS WORKING IN TRACTION

(30) Priorité: 13.06.2006 FR 0605236
(43) Date de publication de la demande: 25.02.2009
(73) Titulaire: Soubeiran, Arnaud, 75014 Paris (FR)
(72) Inventeur: Soubeiran, Arnaud, 75014 Paris (FR)
(74) Mandataire: Demulsant, Xavier
(86) Numéro de dépôt international: PCT/FR2007/000950
(87) Numéro de publication internationale: WO 2007/144489

(56) Documents cités:
- WO-A-01/78614
- WO-A-99/51160
- WO-A-2004/019796
- DE-U1- 8 515 687
- US-A1- 2005 261 779
- US-B1- 6 336 929

## Description

### Domaine Technique

La présente invention concerne les dispositifs d'allongement intracorporels, tels que clous d'allongement osseux, clous de transport osseux, tiges de distraction rachidienne ou prothèses de croissance.

### Technique Antérieure

Plusieurs dispositifs d'allongement intracorporels dont, notamment, ceux décrits dans les documents US 3 976 060, Proc Inst Mech Eng [H]. 1989;203(2):97-102., WO 01/78614, DE 85 15 687 U1 ou US 6 336 929 comprennent une première partie allongée, une seconde partie montée télescopique par rapport à ladite première partie, des premiers moyens de liaison à l'organisme à une première extrémité de ladite première partie, des seconds moyens de liaison à l'organisme à une première extrémité de ladite seconde partie, une tige comportant au moins un filetage dont la rotation entraîne le déplacement de ladite seconde partie par rapport à ladite première partie, des moyens pour commander la rotation de ladite tige et des moyens de liaison entre ces constituants. Toutefois, dans la totalité de ces dispositifs connus ladite tige est montée entre ladite première extrémité de ladite première partie et la seconde extrémité de ladite seconde partie opposée à ladite première extrémité de ladite seconde partie. Ladite tige est ainsi montée entre deux extrémités qui s'éloignent quand le dispositif s'allonge, la longueur de tige chargée augmente quand le dispositif s'allonge et la longueur de tige chargée travaille en compression quand le dispositif s'allonge. De ce fait, la longueur de l'allongement est limitée par la hauteur de flambage de ladite tige sous la charge subie, le volume consacré dans ledit dispositif à ladite tige et au taraudage avec lequel elle coopère qui ne contribue pas à la solidité, notamment en flexion, desdits dispositifs est important et surtout, le couple nécessaire à la mise en rotation de ladite tige est également important, ce qui impose le dimensionnement des moyens pour commander la rotation de ladite tige en conséquence et limite l'usage dans la pratique de certains moyens simples et fiables mais d'une puissance limitée comme la transmission magnétique directe sans moyens d'amplification entre deux aimants permanents par exemple tel que décrit dans le document US 6 336 929.

Le préambule de la revendication 1 ci-après est basé sur le contenu de ce document.

### Exposé de l'invention

La présente invention propose de remédier à ces inconvénients. En effet, dans le dispositif d'allongement intracorporel suivant la présente invention qui comprend une première partie allongée, une seconde partie montée télescopique par rapport à ladite première partie, des premiers moyens de liaison à l'organisme à une première extrémité de ladite première partie, des seconds moyens de liaison à l'organisme à une première extrémité de ladite seconde partie, une tige comportant au moins un filetage dont la rotation entraîne le déplacement de ladite seconde partie par rapport à ladite première partie, des moyens pour commander la rotation de ladite tige et des moyens de liaison entre ces constituants, ladite tige est montée entre deux extrémités qui se rapprochent quand ledit dispositif s'allonge, la seconde extrémité de ladite première partie opposée à ladite première extrémité de ladite première partie et la seconde extrémité de ladite seconde partie opposée à ladite première extrémité de ladite seconde partie.

Ainsi, la longueur de tige chargée diminue quand le dispositif s'allonge et la longueur de tige chargée travaille en traction quand le dispositif s'allonge.

Les moyens de liaison entre ladite tige et ladite seconde extrémité de ladite première partie et les moyens de liaison entre ladite tige et ladite seconde extrémité de ladite seconde partie pouvant comporter respectivement:
- un pivot et un taraudage,
- un taraudage et un pivot,
- un premier taraudage dans un premier sens et un second taraudage dans le sens opposé au dit premier sens, dans ce cas ladite tige comporte un premier filetage apte à coopérer avec ledit premier taraudage à une extrémité et un second filetage apte à coopérer avec ledit second taraudage à l'autre extrémité.

En outre, suivant la présente invention, lesdits moyens pour commander la rotation de ladite tige peuvent être de tous types, mais sont préférentiellement constitués d'un aimant permanent récepteur monté solidaire de ladite tige de manière à ce que sa direction d'aimantation soit sensiblement perpendiculaire à l'axe de rotation de ladite tige et, à l'extérieur de l'organisme, d'une source de champ magnétique d'entraînement tournant sensiblement autour de l'axe de ladite tige. Avantageusement ledit aimant permanent récepteur est un aimant néodyme apte à supporter les températures de stérilisation dudit dispositif d'allongement intracorporel suivant la présente invention et ladite source de champ magnétique d'entraînement tournant à l'extérieur de l'organisme est au moins un aimant néodyme que l'on entraîne en rotation sensiblement autour de l'axe de ladite tige en maintenant un de ses pôles constamment tourné vers elle. Tout aussi avantageusement, la vitesse de rotation dudit champ magnétique pourra être inférieure à un tour par minute. Le diamètre de ladite tige sera quant à lui avantageusement inférieur à 4mm.

### Description sommaire des dessins

L'invention, son fonctionnement et ses applications seront mieux compris et d'autres de ses caractéristiques et avantages apparaîtront au cours de la description suivante donnée en regard des dessins annexés à titre illustratif mais nullement limitatif dans lesquels :
Les figures 1 à 3 représentent un mode de réalisation préféré du dispositif suivant l'invention plus particulièrement utile pour l'allongement de membres. La figure 1 est une vue éclatée en perspective avec les lignes cachées en pointillés de ce mode de réalisation. La figure 2 en est une vue en coupe passant par l'axe de ladite tige comportant au moins un filetage dans la position la plus courte dudit mode de réalisation.
La figure 3 est une vue en coupe passant toujours par l'axe de ladite tige où ledit mode de réalisation est représenté presque totalement allongé.

Il est bien précisé que, sur les figures, les mêmes références désignent les mêmes éléments, quelle que soit la figure sur laquelle ils apparaissent et quelle que soit la forme de représentation de ces éléments. De même, si des éléments ne sont pas spécifiquement référencés sur l'une des figures, la référence peut être aisément retrouvée en se reportant à une autre figure. Dans toutes les figures, les dimensions et proportions ont été adaptées quand cela pouvait faciliter la compréhension. En outre, lorsque que, selon la définition de l'invention, l'objet de l'invention comporte « au moins un » élément ayant une fonction donnée, le mode de réalisation décrit peut comporter plusieurs de ces éléments.

### Manières de réaliser l'invention

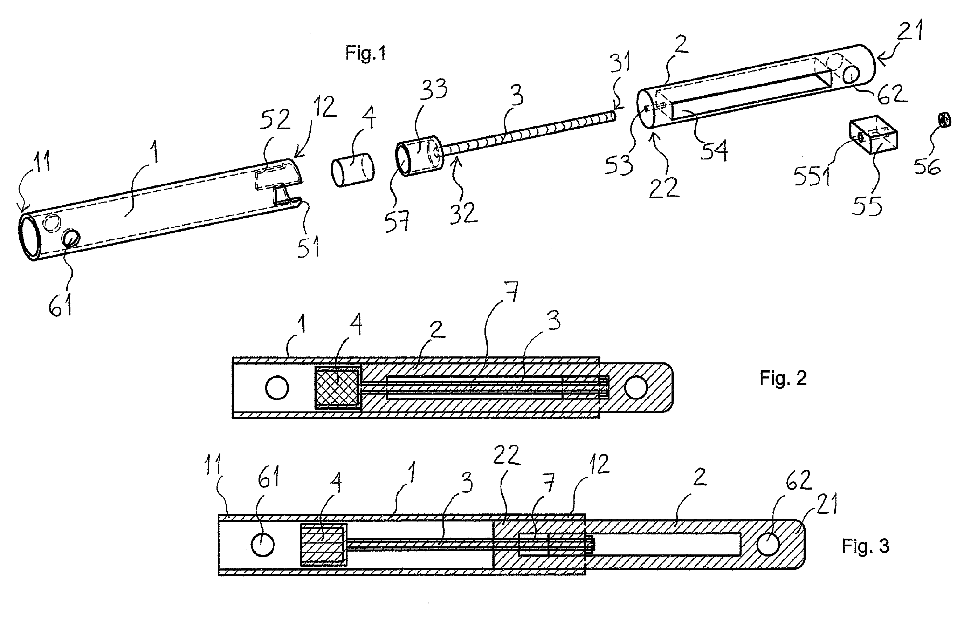

Le mode de réalisation préféré du dispositif représenté sur les figures 1 à 3 comporte :
- une première partie (1) allongée sensiblement tubulaire qui comporte, à une première extrémité (11), des moyens de liaison à l'organisme, constitués par exemple par un premier perçage (61) sensiblement perpendiculaire à l'axe de ladite première partie (1) et destiné à recevoir une vis, non représentée, et à la seconde extrémité (12), opposée à ladite première extrémité (11), deux entailles longitudinales et diamétralement opposées (51),(52). Ladite première extrémité (11) peut également comporter des moyens de préhension non représentés, tel qu'un filetage est une encoche par exemple, dudit dispositif pour faciliter son implantation chirurgicale, en général dans le canal médullaire d'un os long ou le long ou dans le prolongement de tout ou partie d'un os long ou plat, tout comme son explantation.
- une seconde partie (2) sensiblement cylindrique dont le diamètre extérieur correspond sensiblement au diamètre intérieur de ladite première partie (1) avec laquelle elle coopère télescopiquement et qui comporte, à une première extrémité (21), des moyens de liaison à l'organisme, constitués par exemple par un deuxième (62) perçage sensiblement perpendiculaire à l'axe de ladite seconde partie (2) et destiné à recevoir une vis, non représentée, et à la seconde extrémité (22), opposée à ladite première extrémité (21), et qui reste toujours en contact avec ladite première partie (1), un taraudage (53) sensiblement suivant l'axe de ladite seconde partie (2). Entre les deux, une rainure (54) longitudinale et débouchant diamétralement de part et d'autres de ladite seconde partie (2). La largeur de ladite rainure (54) étant sensiblement égale à la largeur desdites entailles (51),(52) pratiquées dans ladite seconde extrémité (12) de ladite première partie (1) et ledit taraudage (53) débouchant dans ladite rainure (54) et ayant un diamètre sensiblement inférieur à la largeur de ladite rainure (54).

- une tige (3) qui comporte à une première extrémité (31) un filetage de diamètre et de pas égaux à ceux dudit taraudage (53) de ladite seconde partie (2) avec lequel ledit filetage coopère,
- des moyens de liaison entre ces constituants constitués:
   - d'une languette d'appui (55) sensiblement parallélépipédique et apte à se loger dans lesdites deux entailles (51), (52) pratiquées dans ladite seconde extrémité (12) de ladite première partie (1) de manière à former un pont entre elles. Ladite languette d'appui (55) comportant en outre sensiblement en son milieu un perçage (551) dans lequel ladite première extrémité (31) de ladite tige (3) peut coulisser et tourner librement,
   - d'un écrou d'appui (56) apte à se visser sur ladite première extrémité (31) de la tige (3). Ladite languette d'appui (55) et ledit écrou d'appui (56) constituant un pivot entre ladite tige (3) et ladite seconde extrémité (12) de ladite première partie (1).

Dans ce mode préféré de réalisation de l'invention, lesdits moyens pour commander la rotation de ladite tige (3) sont constitués:
- d'un aimant permanent récepteur cylindrique (4) à aimantation diamétrale solidarisé coaxialement à ladite tige (3) par exemple par collage à l'aide d'une colle silicone bien tolérée par l'organisme dans une cavité cylindrique (57) coaxiale à l'axe de ladite tige (3) et pratiquée dans un renflement cylindrique (33) solidaire de la seconde extrémité (32) opposée à ladite première extrémité (31) de ladite tige (3).
- d'une source de champ magnétique d'entraînement tournant autour de l'axe de ladite tige (3) et non représentée sur les figures 1 à 3 placée à l'extérieur de l'organisme.

On note que lesdits moyens pour commander la rotation de ladite tige (3) peuvent également être constitués de tout autre moyen connu de l'homme de l'art tels qu'un moteur électrique avec ou sans réducteur et une source de courant interne ou externe à l'organisme ou un aimant permanent avec réducteur et une source de champ magnétique tournant externe à l'organisme ou encore un système de roues libres apte à transformer les couples qui peuvent exister entre ladite première partie (1) et ladite seconde partie (2) du dispositif quand le patient bouge en rotation dans un seul sens de ladite tige (3). Ces dits moyens pouvant être aptes à entraîner ladite tige (3) dans un sens seulement ou dans les deux sens suivant le besoin.

Les différents constituants dudit mode de réalisation préféré du dispositif représenté sur les figures 1 à 3 s'assemblent de la manière suivante : ladite première extrémité (31) de ladite tige (3) est vissée dans ledit taraudage (53) de ladite seconde partie (2). Dés que ladite première extrémité (31) de ladite tige (3) dépasse suffisamment dans ladite rainure (54) de ladite seconde partie (2), ladite languette d'appui (55) est glissée dans ladite rainure (54) puis enfilée, à travers son perçage (551), sur ladite première extrémité (31) de ladite tige (3). L'écrou d'appui (56) est alors vissé sur ladite première extrémité (31) de ladite tige (3) qui dépasse de ladite languette d'appui (55) dans ladite rainure (54) et bloqué définitivement sur ladite tige (3) par exemple au moyen d'une soudure laser en bout, ou d'un écrasement à travers ladite rainure (54) entraînant une ovalisation d'une partie au moins dudit écrou (56) et de la portion de ladite première extrémité (31) de ladite tige (3) qui s'y trouve. L'ensemble ainsi constitué est introduit dans ladite seconde extrémité (12) de ladite première partie (1), ladite seconde extrémité (32) de ladite tige (3) et ladite seconde extrémité (22) de ladite seconde partie (2) en premier, jusqu'à ce que ladite languette d'appui (55) vienne en butée dans lesdites entailles (51), (52) de ladite seconde extrémité (12) de ladite première partie (1). On peut alors solidariser définitivement ladite languette d'appui (55) et ladite première partie (1) par exemple au moyen d'une soudure laser. L'assemblage de ce mode préféré de réalisation de l'invention est ainsi achevé.

Toutes les pièces sont réalisées en matériau mécaniquement résistant et bien toléré par l'organisme tels que 316L refondu sous vide, certains alliages de titane, ou alliages haute résistance à base de chrome et de cobalt. Avantageusement les pièces, et particulièrement ladite tige (3), sont traitées en surface pour limiter les frottements et l'usure éventuelle. Des traitements à base de carbone amorphe diamantin ou de bisulfure de tungstène sont préférés. La combinaison du faible diamètre de ladite tige (3) permis par son montage et son travail en traction et du bas coefficient de friction obtenu grâce aux dits traitements de surface permet l'entraînement de ladite tige (3) par un couple modeste au regard de la charge appliquée et de ce qu'il serait si ladite tige (3) avait été dimensionnée pour un travail en compression. Le diamètre de ladite tige (3) est couramment compris entre un et trois millimètres et ne dépasse pas quatre millimètres pour une prothèse de patient adulte par exemple.

Si la tige (3) a été prévue de longueur suffisante, le potentiel de variation de longueur maximal dudit mode de réalisation préféré dudit dispositif suivant l'invention est sensiblement égal à la longueur de ladite rainure (54) de ladite seconde partie (2) diminuée de la longueur nécessaire au logement de ladite languette d'appui (55) et dudit écrou d'appui (56), sinon c'est la longueur de ladite tige (3) qui limite ledit potentiel.

Le fonctionnement de ce mode préféré de réalisation de l'invention est évident : en présence d'un champ magnétique d'entraînement perpendiculaire à l'axe de ladite tige (3) et tournant autour de cet axe, ledit aimant permanent récepteur cylindrique (4) solidarisé coaxialement à ladite tige (3) tend à s'orienter dans ledit champ magnétique d'entraînement appliquant un couple sur ladite tige (3) et provoquant sa rotation dans le sens de la rotation dudit champ magnétique d'entraînement si ce couple est supérieur au couple résistant de ladite tige (3) sous la charge qu'elle subit au moment de l'activation dudit champ magnétique d'entraînement. Ladite rotation de ladite tige (3) se transforme en déplacement dans un sens ou dans l'autre de ladite seconde partie (2) par rapport à ladite première partie (1) suivant le sens du pas des dits tige (3) et taraudage (53) et ainsi en allongement ou en raccourcissement dudit dispositif suivant l'invention. Ladite seconde extrémité (12) de ladite première partie (1) et ladite seconde extrémité (22) de ladite seconde partie (2) se rapprochent quand le dispositif s'allonge. La longueur chargée (7) de ladite tige (3) diminue quand le dispositif s'allonge et ladite longueur chargée (7) l'est en traction quand le dispositif s'allonge.

En outre, toujours en se référant audit mode de réalisation préféré du dispositif suivant l'invention représenté sur les figures 1 à 3, il est impossible d'appliquer une compression sur ladite tige (3) car elle coulisse alors dans le perçage (551) de ladite languette d'appui (55), l'écrou d'appui (56) ne la bloquant que dans l'autre sens, autre sens dans lequel la tige (3) subit une traction.

La création d'un champ magnétique d'entraînement propice à la mise en rotation dudit aimant permanent récepteur (4) cylindrique solidarisé coaxialement à ladite tige (3) et de ladite tige (3) peut être réalisée par tout moyen, mais préférentiellement en disposant à l'extérieur de l'organisme, à la hauteur dudit aimant permanent récepteur (4) au moins un autre aimant permanent d'entraînement dont l'un des pôles sera tourné vers l'axe commun à ladite tige (3) et audit aimant permanent récepteur (4). La combinaison de deux aimants permanents d'entraînement disposés de manière à ce que le pôle sud de l'un face face au pôle nord de l'autre et que ledit aimant permanent récepteur (4) et la partie de l'organisme qui entoure ledit dispositif soient placés entre les deux produira un couple d'entraînement encore supérieur et pourra être préféré.

Il est bien précisé que le mode de réalisation de l'invention représenté sur les figures 1 à 3 est un mode préféré de réalisation de l'invention mais que d'autres modes de réalisation répondant à la définition de l'invention existent et pourront être réalisés par l'homme de l'art. En particulier ladite tige (3) pourra être excentrée par rapport aux dites première (1) et seconde (2) pièces et lesdites première (1) et seconde (2) pièces pourront présenter des géométries différentes d'un tube et d'un cylindre respectivement et être chacune constituées de plusieurs parties assemblées par tout moyen. En outre, ladite première extrémité (21) de ladite seconde partie (2) pourra être du côté de ladite première extrémité (11) de ladite première partie (1) et ladite seconde extrémité (22) de ladite seconde partie (2) du côté de ladite seconde extrémité (12) de ladite première partie (1), à l'inverse du mode préféré de réalisation l'invention représentée sur les figures 1 à 3. dans cette dernière configuration, ladite seconde partie (2) est constamment entièrement en contact avec ladite première partie (1) et des gorges sont pratiquées dans ladite première partie (1) pour permettre la liaison vers les moyens de liaison à l'organisme que comporte ladite première extrémité (21) de ladite seconde partie (2) sur toute la course d'allongement dudit dispositif.

### Possibilités d'application industrielle

La présente invention est particulièrement utile pour la réalisation de tout type de dispositif d'allongement intracorporel et particulièrement de clous d'allongement médullaires et de prothèses de croissance d'os longs.

## Revendications

1. Dispositif d'allongement intracorporel comprenant une première partie allongée (1), une seconde partie (2) montée télescopique par rapport à ladite première partie (1), des premiers moyens de liaison (61), à l'organisme à une première extrémité (11) de ladite première partie (1), des seconds moyens de liaison (62), à l'organisme à une première extrémité (21) de ladite seconde partie (2), une tige (3) comportant au moins un filetage dont la rotation entraîne le déplacement de ladite seconde partie (2) par rapport à ladite première partie (1), des moyens (4) pour commander la rotation de ladite tige (3) et des moyens de liaison entre ces constituants **caractérisé en ce que** ladite tige (3) est montée entre deux extrémités qui se rapprochent quand ledit dispositif s'allonge, la seconde extrémité (12) de ladite première partie (1) opposée à ladite première extrémité (11) de ladite première partie (1) et la seconde extrémité (22) de ladite seconde partie (2) opposée à ladite première extrémité (21) de ladite seconde partie (2) de manière à ce que la longueur de la tige chargée travaille en traction quand le dispositif s'allonge.

2. Dispositif d'allongement intracorporel suivant la première revendication **caractérisé en ce que** les moyens de liaison entre ladite tige (3) et ladite seconde extrémité (12) de ladite première partie (1) comportent un pivot (55), (56) et les moyens de liaison entre ladite tige (3) et ladite seconde extrémité (22) de ladite seconde partie (2) comportent un taraudage (53) apte à coopérer avec ladite tige (3).

3. Dispositif d'allongement intracorporel suivant la première revendication **caractérisé en ce que** les moyens de liaison entre ladite tige (3) et ladite seconde extrémité (12) de ladite première partie (1) comportent un taraudage apte à coopérer avec ladite tige (3) et les moyens de liaison entre ladite tige (3) et ladite seconde extrémité (22) de ladite seconde partie (2) comportent un pivot.

4. Dispositif d'allongement intracorporel suivant la première revendication **caractérisé en ce que** les moyens de liaison entre ladite tige (3) et ladite seconde extrémité (12) de ladite première partie (1) comportent un premier taraudage dans un premier sens et les moyens de liaison entre ladite tige (3) et ladite seconde extrémité (22) de ladite seconde partie (2) comportent un second taraudage dans le sens opposé audit premier sens et que ladite tige (3) comporte un premier filetage apte à coopérer avec ledit premier taraudage à une extrémité et un second filetage apte à coopérer avec ledit second taraudage à l'autre extrémité.

5. Dispositif d'allongement intracorporel suivant la première revendication **caractérisé en ce que** lesdits moyens pour commander la rotation de ladite tige (3) sont constitués d'au moins un aimant permanent (4) monté solidaire de ladite tige (3) de manière à ce que sa direction d'aimantation soit sensiblement perpendiculaire à l'axe de rotation de ladite tige (3) et, à l'extérieur de l'organisme, d'une source de champ magnétique tournant sensiblement autour de l'axe de ladite tige (3).

6. Dispositif d'allongement intracorporel suivant la cinquième revendication **caractérisé en ce que** la vitesse de rotation dudit champ magnétique produit par ladite source de champ magnétique tournant est inférieure à un tour par minute.

7. Dispositif d'allongement intracorporel suivant la première revendication **caractérisé en ce que** le diamètre de ladite tige (3) est inférieur à quatre millimètres.

## Patentansprüche

1. Intrakorporale Verlängerungsvorrichtung, umfassend einen ersten verlängerten Teil (1), einen zweiten Teil (2), der teleskopartig bezüglich des ersten Teils (1) montiert ist, erste Mittel (61) zur Verbindung mit dem Organismus an einem ersten Ende (11) des ersten Teils (1), zweite Mittel (62) zur Verbindung mit dem Organismus an einem ersten Ende (21) des zweiten Teils (2), eine Stange (3), die mindestens ein Gewinde umfasst und deren Drehung die Verschiebung des zweiten Teils (2) bezüglich des ersten Teils (1) antreibt, Mittel (4) zur Steuerung der Drehung der Stange (3) und Verbindungsmittel zwischen diesen Bestandteilen, **dadurch gekennzeichnet, dass** die Stange (3) zwischen zwei Enden montiert ist, die sich aneinander annähern, wenn sich die Vorrichtung verlängert, wobei das zweite Ende (12) des ersten Teils (1) dem ersten Ende (11) des ersten Teils (1) gegenüberliegt und das zweite Ende (22) des zweiten Teils (2) dem ersten Ende (21) des zweiten Teils (2) gegenüberliegt, so dass die Länge der beaufschlagten Stange auf Zug arbeitet, wenn sich die Vorrichtung verlängert.

2. Intrakorporale Verlängerungsvorrichtung nach dem ersten Anspruch, **dadurch gekennzeichnet, dass** die Verbindungsmittel zwischen der Stange (3) und dem zweiten Ende (12) des ersten Teils (1) einen Drehzapfen (55), (56) umfassen und die Verbindungsmittel zwischen der Stange (3) und dem zweiten Ende (22) des zweiten Teils (2) ein Innengewinde (53) umfassen, das zum Zusammenwirken mit der Stange (3) geeignet ist.

3. Intrakorporale Verlängerungsvorrichtung nach dem ersten Anspruch, **dadurch gekennzeichnet, dass** die Verbindungsmittel zwischen der Stange (3) und dem zweiten Ende (12) des ersten Teils (1) ein Innengewinde umfassen, das zum Zusammenwirken mit der Stange (3) geeignet ist, und die Verbindungsmittel zwischen der Stange (3) und dem zweiten Ende (22) des zweiten Teils (2) einen Drehzapfen umfassen.

4. Intrakorporale Verlängerungsvorrichtung nach dem ersten Anspruch, **dadurch gekennzeichnet, dass** die Verbindungsmittel zwischen der Stange (3) und dem zweiten Ende (12) des ersten Teils (1) ein erstes Innengewinde in einer ersten Richtung umfassen und die Verbindungsmittel zwischen der Stange (3) und dem zweiten Ende (22) des zweiten Teils (2) ein zweites Innengewinde in der der ersten Richtung entgegengesetzten Richtung umfassen und dass die Stange (3) ein erstes Außengewinde, das geeignet ist, mit dem ersten Innengewinde an einem Ende zusammenzuwirken, und ein zweites Außengewinde, das geeignet ist, mit dem zweiten Innengewinde am anderen Ende zusammenzuwirken, umfasst.

5. Intrakorporale Verlängerungsvorrichtung nach dem ersten Anspruch, **dadurch gekennzeichnet, dass** die Mittel zur Steuerung der Drehung der Stange (3) aus mindestens einem Permanentmagneten (4), der fest an der Stange (3) montiert ist, so dass seine Magnetisierungsrichtung im Wesentlichen senkrecht zur Rotationsachse der Stange (3) verläuft, und außerhalb des Organismus aus einer Magnetfeldquelle bestehen, die sich im Wesentlichen um die Achse der Stange (3) dreht.

6. Intrakorporale Verlängerungsvorrichtung nach dem fünften Anspruch, **dadurch gekennzeichnet, dass** die Drehzahl des von der sich drehenden Magnetfeldquelle erzeugten Magnetfelds unter einer Umdrehung pro Minute liegt.

7. Intrakorporale Verlängerungsvorrichtung nach dem ersten Anspruch, **dadurch gekennzeichnet, dass** der Durchmesser der Stange (3) kleiner als vier Millimeter ist.

## Claims

1. Intra-corporal expandable device comprising a first elongated part (1), a second part (2) assembled telescopically relative to the said first part (1), first links (61) to the organism at a first end (11) of the said first part (1), second links (62) to the organism at a first end (21) of the said second part (2), a rod (3) with at least one thread, the rotation of which leads to the displacement of the said second part (2) relative to the said first part (1), means (4) for controlling the rotation of the said rod (3) and links between these components **characterized in that** the said rod (3) is assembled between two ends which come closer when the said device is elongated, the second end (12) of the said first part (1) opposite the said first end (11) of the said first part (1) and the second end (22) of the said second part (2) opposite the said first end (21) of the said second part (2) so that the loaded rod length operates under traction when the device is elongated.

2. Intra-corporal expandable device according to Claim 1, **characterized in that** the links between the said rod (3) and the said second end (12) of the said first part (1) comprise a pivot (55), (56) and the links between the said rod (3) and the said second end (22) of the said second part (2) comprise a tapping (53) able to cooperate with the said rod (3).

3. Intra-corporal expandable device according to Claim 1, **characterized in that** the links between the said rod (3) and the said second end (12) of the said first part (1) comprise a tapping able to cooperate with the said rod (3) and the links between the said rod (3) and the said second end (22) of the said second part (2) comprise a pivot.

4. Intra-corporal expandable device according to Claim 1, **characterized in that** the links between the said rod (3) and the said second end (12) of the said first part (1) comprise a first tapping in a first direction and the links between the said rod (3) and the said second end (22) of the said second part (2) comprise a second tapping in the direction opposite to the said first direction and that the said rod (3) comprises a first thread able to cooperate with the said first tapping at one end, and a second thread able to cooperate with the said second tapping at the other end.

5. Intra-corporal expandable device according to Claim 1, **characterized in that** the said means to control the rotation of the said rod (3) are comprised of at least one permanent magnet (4) assembled coupled with the said rod (3) such that its magnetisation direction is roughly perpendicular to the axis of rotation of the said rod (3) and, outside the organism, of a magnetic field source rotating roughly around the axis of the said rod (3).

6. Intra-corporal expandable device according to Claim 5, **characterized in that** the speed of rotation of the said magnetic field produced by the said rotating magnetic field source is less than one revolution per minute.

7. Intra-corporal expandable device according to Claim 1, **characterized in that** the diameter of the said rod (3) is less than four millimetres.
